Europäisches Patentamt

European Patent Office

Office européen des brevets

(19)

(11) Publication number: **0 199 876**
**A2**

(12) **EUROPEAN PATENT APPLICATION**

(21) Application number: **85308855.7**

(22) Date of filing: **05.12.85**

(51) Int. Cl.⁴: **A 61 M 1/00**
**A 61 B 1/12**

(30) Priority: **19.04.85 US 725691**

(43) Date of publication of application:
**05.11.86 Bulletin 86/45**

(84) Designated Contracting States:
**CH DE FR GB LI**

(71) Applicant: **Warner-Lambert Technologies, Inc.**
**201 Tabor Road**
**Morris Plains New Jersey 07950(US)**

(72) Inventor: **Brunell, Robert A.**
**4 Bellevue Avenue**
**South Bridge Massachusetts 01550(US)**

(74) Representative: **Coxon, Philip et al,**
**Eric Potter & Clarkson 14 Oxford Street**
**Nottingham NG1 5BP(GB)**

(54) Valve system for medical and veterinary appliances.

(57) An improved valve device (10) useful in endoscopes, bronchoscopes, laryngoscopes and the like in medical or veterinary examinations facilitating the selective application of suction or irrigation fluids (to effect a lavage procedure) at the distal end of the scope without interrupting or disturbing the continuity and integrity of the examination procedure. The valve device (10) includes a first outlet (12) connectible to a scope shaft, a second outlet (13) connectible to a source of suction and a third outlet (14) connectible to a fluid pump (S). Valve means (30) is disposed between the fluid pump (S) and the first outlet (12) to control fluid flow from the fluid pump (S).

Fig. 1

EP 0 199 876 A2

Croydon Printing Company Ltd.

-1-

## DESCRIPTION

The present invention relates to a valve device and deals in particular with a valve device useful in endoscopes, bronchoscopes, laryngoscopes and the like in veterinary and medical applications.

In such procedures or examinations, it is frequently necessary and desirable to irrigate (effect a lavage procedure) and/or evacuate the region surrrounding the distal end of the scope under examination.

In prior art valve devices, the shifting from lavage to a suction mode requires, invariably, an interruption of the continuity of the examination in order to make cumbersome connections. That is, the suction must be terminated or bypassed to an outlet to atmosphere and a pump device must be physically inserted into the system. Usually the pump device must be removed before suction can again be applied.

A prior art device over which the present invention is an improvement is shown and described in U.S. Patent No. 3,958,566 issued May 25, 1976 to Hiroyuki Furihata, and assigned on its face to Olympus Optical Co., Ltd., Tokyo, Japan.

In this specification a suction pipe 26 communicates with a channel 14 through a port 22. Normally the suction vents to atmosphere through a tube 34 (Fig. 2) or an opening 25a (Figs. 10 & 11).

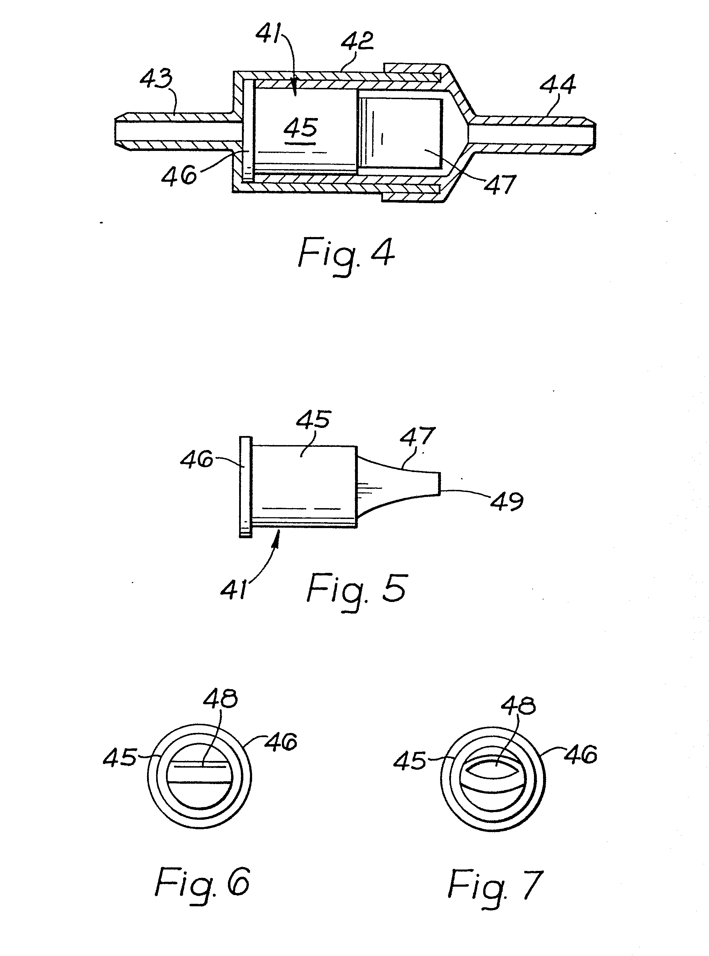

When it is desired to irrigate or convert to the lavage mode, it is necessary physically to insert a syringe (injection instrument 38, Figs. 5, 7 and 8) into a bell mouth 17a cutting off the passage 22 to suction as the sliding tube 34 is depressed (Figs. 7, 10 and 11) against a biasing spring 21.

In contrast to the prior art, the present invention makes is possible to have fluid for irrigation or suction immediately available to the user of the scope in that the syringe is always attached in place. Irrigation and suction can be actuated selectively as desired without the need for cumbersome manipulative procedures.

Thus, with the present invention the user of the scope can shift from suction to lavage mode, and vice versa, by conveniently fingering the appropriate elements of the valve system.

According to the present invention there is provided a valve device comprising a valve body, a first outlet in said body adapted to connect to a scope shaft, a second outlet in said body communicating with said first outlet and adapted to make a connection with a source of suction, a third outlet in said body communicating with said first outlet and adapted to make a connection with a fluid pump, and a spool valve disposed in a bore in said body movable from a first or normal position to vent the suction to atmosphere and connect the fluid pump to the scope shaft to a second position to connect the suction to the shaft and close the connection between the fluid pump and the shaft.

Advantageously valve means is disposed between the fluid pump and the first outlet and is operable to seal tightly when the spool valve is in said second position in response to residual suction. Advantageously also, the valve means is operable to open when the spool valve is in said first position in response to fluid pressure from the fluid pump.

In an especially preferred embodiment the valve means is a duckbill valve. Desirably the bill portion of the valve is disposed to project towards the first outlet.

The duckbill valve may conveniently be provided between the third outlet and the fluid pump, in which case the bill portion may be arranged to project towards the third outlet.

Preferably the duckbill valve is contained within a housing.

The valve means according to the invention is especially useful because it does not open in response to residual suction and hence prevents the syringe plunger from being actuated to inject syringe fluid when no fluid is desired. However the valve means will permit fluid to be injected in response to pressure applied to the plunger.

Reference is now made to the accompanying drawings in which:-

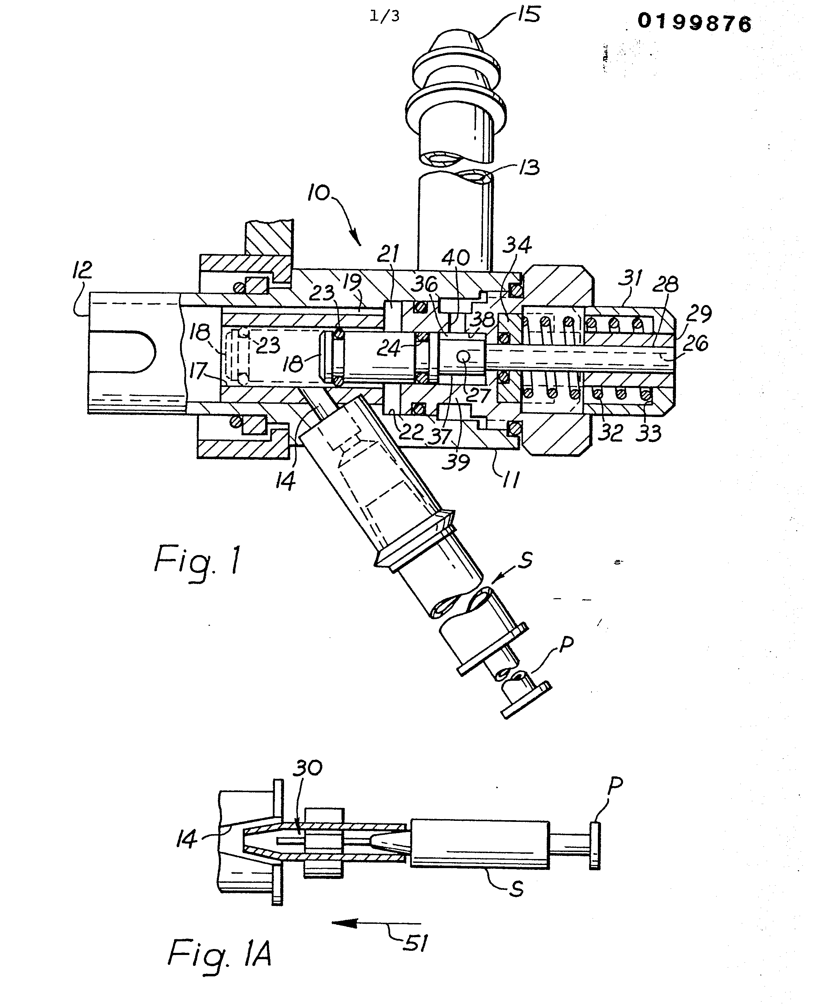

Figure 1 is a sectional view of a valve device according to the invention;

Figure 1A shows, schematically, a normally closed duckbill valve disposed between the valve device of Figure 1 and a syringe;

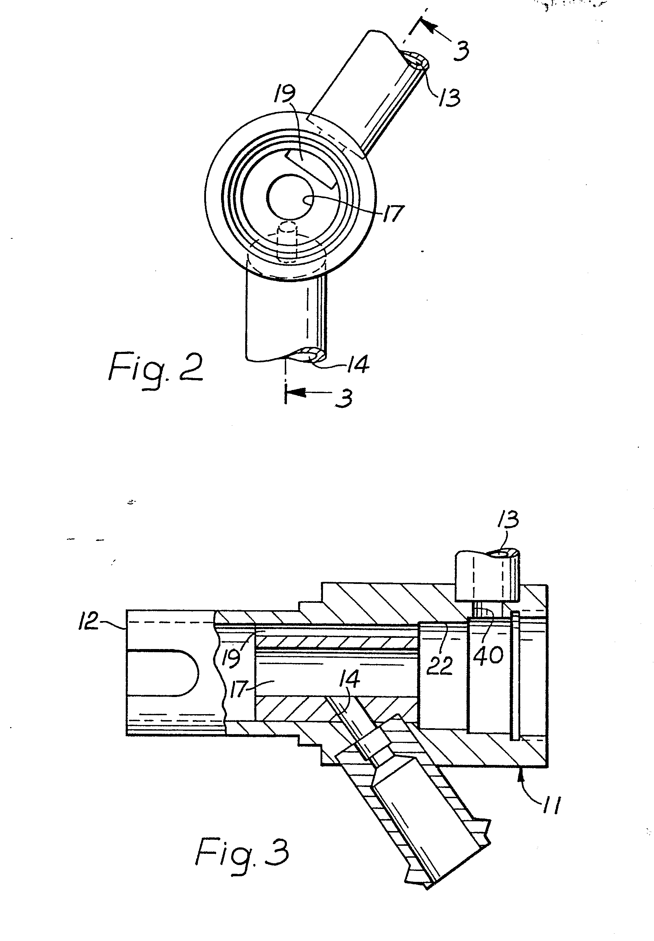

Figures 2 is a view of the right end of Figure 1 with certain parts removed for clarity;

Figure 3 is a sectional view of Figure 2 along intersecting centerlines labelled 3-3;

Figure 4 shows a side view of the duckbill valve of Figure 1A encased in a transparent housing and enlarged for clarity;

Figure 5 is a side elevation (rotated 90°) of the duckbill valve, per se; and

Figures 6 and 7 are views of the right side of Figure 5 showing respectively the closed and open condition of the bill of the valve.

In the drawings a valve device generally designated 10 includes a valve body 11, a first outlet 12 having a bayonet fitting to connect a scope shaft (not shown) in a known manner, a second outlet 13 having a nipple 15 for connecting with a source of vacuum or suction (not shown), and a third outlet 14 adapted to receive and support a fluid pump such as a syringe S.

The body 11 is formed with a central bore 17 receiving a slidable spool valve 18. The bore 17 communicates directly with the shaft through first outlet 12 and with the syringe S through third outlet 14.

The body 11 of the valve device includes an auxiliary passage or conduit 19 which connects with the first outlet 12 and an annulus 21 defined by the periphery of the spool valve 18 and the undercut 22 in the body 11 as is most apparent in Figure 1.

The spool valve 18, which is fitted with two spaced seal means defining O-rings 23 and 24, is formed with a central bore 26 communicating with a transverse opening 27 and terminates in a stem 28.

The stem 28 makes a press-fit with a sleeve 29 which is in turn secured to operating button 31. Coil spring 32, seating in the button 31 as at 33 and at a gland 34, is under a compressive stress sufficient to retain the spool valve 18 in the first or normal position as shown in solid lines in Figure 1.

The button 31 is depressible manually against the bias of spring 32 to move the spool valve 18 to the operated or second position indicated by dotted lines in Figure 1.

In this operated position of the spool valve 18, the first outlet 12 communicates with the source of suction through auxiliary passage 19, annulus 36 (defined by undercut 37 in spool valve 18 and internal surface 38 of a gland 39) and aperture 40 (see Figure 3). Note that in the operated position the O-rings 23 and 24 straddle outlet 14 to seal off the syringe S.

The operation of the valve device 10 will now be described.

Assume that the valve device 10 is in the normal condition as illustrated in solid lines in Figure 1. Assume further that a fluid pump such as syringe S is fixed in position in valve body 11, and the valve body is connected to a scope shaft and to a source of suction.

With the spool valve 18 in the solid line position (first position), suction bypasses to atmosphere from outlet 13, through aperture 40, annulus 36, transverse opening 27 and bore 26.

If, at this time, it is desirable to activate a lavage procedure, the scopist merely depresses the plunger P of the syringe S without interrupting the continuity of the examination being observed at the distal end of shaft. The suction also remains continuous.

When it is desired to introduce suction to the shaft, the user merely depresses the button 31 manually with an appropriate digit, making certain to cover the bore 26, closing it to the atmosphere.

Depression of the button 31 moves spool valve 18 to the second or dotted line position of Figure 1. The O-rings 23 and 24 seal the third outlet 14 to the syringe S and connect the suction to the first outlet 12 through auxiliary passage 19, as previously described.

The outlet 14 is sealed because O-rings 23 and 24 straddle the outlet 14 as shown in dotted lines in Figure 1.

To avoid siphoning action through the syringe S when the spool valve 18 is in the second position, particularly when the suction is at a high level, it is desirable to include valve means in the form of a duckbill valve and a housing between the outlet 14 and the syringe S. The valve means is generally designated 30 in Figure 1A. As will be apparent shortly, the duckbill valve is positioned so that the bill portion is downstream, i.e., projecting toward outlet 14.

Referring to Figures 4 through 7, a duckbill valve 41, encased in a housing 42 having an inlet 43 and an outlet 44, is fabricated of flexible, rubber-like material.

A hollow tubular body 45 terminates at one end in a flange 46 and at the opposite end in a "bill" 47.

The bill is formed with an offset, through slit 48 providing a passage extending from the front 49 of the bill to the interior of the hollow tubular body. The bill 47 is generally flat and normally collapsed and sealed as shown in Figure 6.

When the spool valve 18 is in the solid line position (continuous suction venting to the atmosphere through bore 26), depression of the plunger of syringe S in the direction of the arrow 51 of Figure 1A introduces fluid into the system via the third outlet 14. During this occurrence, the bill 47 is blown open, i.e., the slit 48 opens as shown in Figure 7, allowing fluid to pass through the valve.

When the spool valve 18 is in the dotted line position (suction drawn on the outlet 12), any residual suction or vacuum that may leak by O-rings 23 and 24 will operate to maintain the collapse of the bill 47 keeping the slit 48 sealed thereby avoiding undesirable "drawndown" of fluid from the syringe.

Thus, when fluid pressure is applied by the syringe S, the bill 47 is blown open to permit fluid to flow to the third outlet 14; when no fluid pressure is applied by the syringe, the presence of suction or vacuum in the outlet 44 acts to pull the bill closed in a positive manner.

It is apparent that the present invention provides a valve device which facilitates the selective application of irrigation fluid and suction in scopes of the class described with no need to shut down an examination or other procedure to change or replace fittings. The invention also facilitates the convenience of continuous suction throughout the time period of a procedure.

CLAIMS

1. A valve device for selectively controlling the application of suction or irrigation fluid in medical or veterinary examination units such as endoscopes, bronchoscopes and laryngoscopes, characterised by

a valve body, a first outlet in said body leading to a scope shaft, a second outlet in said body communicating with said first outlet and defining a suction nipple, a third outlet in said body communicating with said first outlet and adapted to make a connection with a fluid pump, a spool valve disposed in a bore in said body movable from a first or normal position to vent the suction to atmosphere and connect the fluid pump to the first outlet, to a second position to connect the suction to the first outlet and close the connection between the fluid pump and the first outlet, and

valve means disposed between the fluid pump and the first outlet operable to seal tightly when the spool valve is in said second position in response to residual suction, and operable to open when the spool valve is in said first position in response to fluid pressure from the fluid pump.

2. A device according to Claim 1 characterised in that the valve means defines a duckbill valve.

3. A valve device for selectively controlling the application of suction or irrigation fluid in medical or veterinary examination units such as endoscopes, bronchoscopes and laryngoscopes, characterised by

a valve body, a first outlet in said body leading to a scope shaft, a second outlet in said body communicating with said first outlet and defining a suction nipple, a third outlet in said body communicating with said first outlet and adapted to make a connection with a fluid pump, a spool valve disposed in a bore in said body movable from a first or normal position to vent the suction to atmosphere and connect the fluid pump to the first outlet, to a second position to connect the suction to the first outlet and close the connection between the fluid pump and the first outlet, and

valve means defining a duckbill valve disposed between the fluid pump and the first outlet.

4. A valve device according to Claim 2 or 3, characterised in that the duckbill valve is disposed between the fluid pump and said third outlet and the bill portion of said valve is disposed so as to project toward said third outlet.

5. A valve device for selectively controlling the application of suction or irrigation fluid in medical or veterinary examination units such as endoscopes, bronchoscopes and laryngoscopes characterised by

a valve body, a first outlet in said body leading to a scope shaft, a second outlet in said body communicating with said first outlet and defining a suction nipple, a third outlet in said body communicating with said first outlet and conformed to make a connection with a fluid pump, a spool valve disposed in a bore in said body movable from a first or normal position to vent suction to atmosphere and to connect the fluid pump to the first outlet to a second position to connect the suction to the first outlet and close the connection between the fluid pump and the first outlet and a valve means disposed between the fluid pump and the first outlet operable to seal tightly when the spool valve is in said second position in response to residual suction, said valve means defining a flexible duckbill valve disposed so that the bill portion thereof projects toward said first and third outlets.

6. A device according to Claim 2, 3, 4 or 5 characterised in that the duckbill valve is contained within a housing.

7. A device according to any preceding claim characterised in that the spool valve carries spaced seal means which in the normal position of the spool valve blocks passage of suction to the shaft outlet and clears access of the fluid pump to the shaft outlet.

8. A device according to any preceding claim characterised in that the body of the valve includes an auxiliary passage making a connection between the third outlet and the first outlet when the spool valve is in the second position.

1/3

0199876

Fig. 1

Fig. 1A

*Fig. 2*

*Fig. 3*

0199876

Fig. 4

Fig. 5

Fig. 6

Fig. 7